## Europäisches Patentamt

## European Patent Office

## Office européen des brevets

(11) Publication number: **0 220 881**
A2

# (12) EUROPEAN PATENT APPLICATION

(21) Application number: 86308009.9

(22) Date of filing: 15.10.86

(51) Int. Cl.⁴: **C 01 B 33/28**
B 01 J 29/28, C 07 C 5/41

(30) Priority: 15.10.85 GB 8525405

(43) Date of publication of application:
06.05.87 Bulletin 87/19

(84) Designated Contracting States:
AT BE CH DE ES FR GB IT LI LU NL SE

(71) Applicant: EXXON CHEMICAL PATENTS INC.
200 Park Avenue
Florham Park New Jersey 07932 (US)

(72) Inventor: Wortel, Theodorus Maria
Mozartlaan 28
NL-3161 RL Rhoon (NL)

Verduijn, Johannes Petrus
Westersingel 34
NL-3202 XL Spijkenisse (NL)

(74) Representative: Northover, Robert Frank et al
ESSO Chemical Limited Esso Chemical Research Centre
P.O. Box 1
Abingdon Oxfordshire, OX13 6BB (GB)

(54) Hexagonal Zeolite L.

(57) Zeolite L in which the cystallites have a hexagonal cross-section, preferably being in the form of well-defined, smooth-surfaced hexagonal rods with a mean diameter of 0.5 to 4 micron and an aspect ratio of from 1 to 5, show increased selectivity to bezene when used as a catalyst substrate on the aromatization of $C_6$ aliphatic hydrocarbons.

EP 0 220 881 A2

**Description**

# HEXAGONAL ZEOLITE L

This invention relates to a highly crystalline zeolite L, its preparation and use in catalysis, particularly for aromatization. In particular, it relates to zeolite L with novel morphology, which provides a catalyst base giving extended lifetimes in the dehydrocyclization of alkanes.

Zeolite L has been known for some time as an adsorbant, and in US 3,216,789 is described as an aluminosilicate of the formula:

$0.9 - 1.3 \ M_{2/n}O:Al_2O_3:5.2 - 6.9 \ SiO_2:yH_2O$

(where M is an exchangeable cation of valence n and y is from 0 to 9) having an x-ray diffraction pattern with the following more significant d(A) values:

$16.1 \pm 0.3$
$7.52 \pm 0.04$
$6.00 \pm 0.04$
$4.57 \pm 0.04$
$4.35 \pm 0.04$
$3.91 \pm 0.02$
$3.47 \pm 0.02$
$3.28 \pm 0.02$
$3.17 \pm 0.01$
$3.07 \pm 0.01$
$2.91 \pm 0.01$
$2.65 \pm 0.01$
$2.46 \pm 0.01$
$2.42 \pm 0.01$
$2.19 \pm 0.01$

The preparation of zeolite L described in US 3,216,789 comprises crystallizing the zeolite from a reaction mixture comprising mole ratios:

$K_2O/(K_2O + Na_2O)$ 0.33 - 1
$(K_2O + Na_2O)/SiO_2$ 0.35 - 0.5
$SiO_2/Al_2O_3$ 10 - 28
$H_2O/(K_2O + Na_2O)$ 15 - 41

The silica to alumina ratio in this reaction mixture is significantly higher than the ratio in the formed zeolite.

British Patent 1,202,511 describes a revised zeolite L preparation using lower proportions of silica in the reaction mixture which comprises mole ratio of reactants as:

$K_2O/(K_2O + Na_2O)$ 0.7 - 1
$(K_2O + Na_2O)/SiO_2$ 0.23 - 0.35
$SiO_2/Al_2O_3$ 6.7 - 9.5
$H_2O/(K_2O + Na_2O)$ 10.5 - 50

The ratio $H_2O/(K_2O + Na_2O + SiO_2 + Al_2O_3)$ is preferably not greater than 6 to give a "dry gel".

US 3,867,512 discloses a preparation of zeolite L from a reaction mixture having a molar composition:

$K_2O/(K_2O + Na_2O)$ 0.3 - 1
$(K_2O + Na_2O)/SiO_2$ 0.3 - 0.6
$SiO_2/Al_2O_3$ 10 - 40
$H_2O/(K_2O + Na_2O)$ 15 - 140

in which the silica source is a gel having at least 4.5 weight percent water and prepared in a particular manner.

L. Wilkosz in Pr Chem 409 (1974) - Chemical Abstracts, Vol. 90 (1979) 573478 describes the preparation of zeolite L from a synthesis sol prepared by treating a solution containing silica, potassium hydroxide and sodium hydroxide with a second solution containing potassium aluminate, potassium hydroxide and sodium hydroxide and crystallizing for 72 hours at 20°C and 122 hours at 100°C. The zeolite L product has a $SiO_2:A_2O_3$ ratio of 6.4:1.

G.V. Tsitsishvilli et al in Doklady Akademii NaukSSSR, Vol. 243, No. 2, pp 438-440 (1978) describe the synthesis of zeolite L from alumina-silica gels containing tributylamine. The gels used had the following molar ratios:

2

| | |
|---|---|
| $SiO_2:Al_2O_3$ | 25 |
| $(K_2O + Na_2O):Al_2O_3$ | 18 |
| $(K_2O + Na_2O):SiO_2$ | 0.72 |
| $H_2O/K_2O + Na_2O$ | 20 |
| $K_2O:Na_2O$ | 0.5 |

Y. Nishiimura in Nippon Kagaku Zasshi 91, 11, 1970. pp 1046-9 describes in general terms zeolite L preparation from a synthesis mixture containing colloidal silica, potassium aluminate and potassium hydroxide having a $SiO_2:Al_2O_3$ ratio of 15 - 25, but exemplifies only two synthesis mixtures having the following ratios of components:

$7K_2O:Al_2O_3:20SiO_2:450H_2O$ and

$8K_2O:Al_2O_3:10SiO_2:500H_2O$

Frety et al in C.R. Acad Sc. Paris, t275, Serie c-1215 describes the electron microscopy examination of zeolite L in which particles were said to be observed in the form of slightly deformed cylinders with very variable dimensions.

US 3,298,780 describes a related zeolite known as UJ prepared from an aqueous reactant solution having a composition, expressed as molar ratios of oxides, corresponding to:

$SiO_2/Al_2O_3$ of from 6 to 30

$R_2/uO/SiO_2$ of from 0.30 to 0.70, and

$H_2O/R_2/uO$ of from 80 to 140

at a temperature between 150°F (65.6°C) and 325°F (162.8°C) until the zeolite crystals are formed. Zeolite UJ is described as having nearly cubic shaped crystals with a crystal size ranging upward from 0.05 micron.

GB 1,393,365 describes a further related zeolite known as AG1 which is prepared by reacting at least one aluminium component, at least one silicon component and at least one alkali metal component, in an aqueous medium, the sole or major silicon component being a water glass having a molar ratio $SiO_2/M_2O$ of 3.5 to 4.0 to give a reaction mixture with oxide molar ratios in one of the following ranges:

| Range 1 | $SiO_2/Al_2O_3$ | 7 - 14 |
| | $(K_2O + NaO_2)/SiO_2$ | 0.25 - 0.85 |
| | $K_2O/(K_2O + Na_2O)$ | 0.75 - 1 |
| | $H_2O/(K_2O + Na_2O)$ | 25 - 160 |

| Range 2 | $SiO_2/Al_2O_3$ | 14 - 20 |
| | $(K_2O + Na_2O)/SiO_2$ | 0.25 - 0.85 |
| | $K_2O/(K_2O + Na_2O)$ | 0.5 - 1 |
| | $H_2O/(K_2O + Na_2O)$ | 25 - 160 |

| Range 3 | $SiO_2/Al_2O_3$ | 20 - 40 |
| | $(K_2O + Na_2O)/SiO_2$ | 0.25 - 1 |
| | $K_2O/(K_2O + Na_2O)$ | 0.4 - 1 |
| | $H_2O/(K_2O + Na_2O)$ | 25 - 160 |

EP0096479 describes zeolite L crystallites in the form of distinct circular cylinders with a mean diameter of at least 0.5 micron and an aspect ratio of at least 0.5, and their preparation in which an alkaline reaction mixture comprising water, a source of silicon and a source of aluminium with a composition falling within the following molar ratios (expressed as oxides):
$M_2O/SiO_2$ 0.22 - 0.36
$H_2O/M_2O$ 25 - 90
$SiO_2/Al_2O_3$ 6 - 15
(wherein M is a cation of valence n, and preferably potassium or a mixture of $K + M^1$ in which $M^1$ is an alkali metal or alkaline earth metal such as sodium, calcium, barium, or rubidium, provided that $K_2/M^1_2O + K_2O)$ is at least 0.7) is heated to a temperature of from at least 75 C and preferably from 100°C to 250°C, more preferably from 120°C to 225°C, to form the desired cylindrical aluminosilicate.

EP0142353 describes a process for the preparation of zeolite L in which an alkaline reaction mixture comprising water, a source of silicon and a source of aluminium with a composition having the following molar ratio (expressed as oxides):
$M_2O/SiO_2$ 0.22 - 0.30
$H_2O/M_2O$ 25 - 45
$SiO_2/Al_2O_3$ 10 - 12
(wherein M is as defined hereinbefore) is heated to a temperature of greater than 150°C for a period long enough to form zeolite L.

EP0142354 describes a process for the preparation of zeolite L in which an alkaline reaction mixture comprising water, a source of silicon and a source of aluminium with a composition having the following molar ratio (expressed in oxides):
$M_2O/SiO_2$ 0.24 - 0.35
$H_2O/M_2O$ 35 - 80
$SiO_2/Al_2O_3$ 5.7 - 7.8
(wherein M is as defined hereinbefore) is heated to a temperature of greater than 200 C to form zeolite L.

Further zeolite L preparations are described in EP0142355, EP0142347, EP0142348 and EP0142349.

It was subsequently found that zeolite L may be used as a catalyst base in aromatization reactions. US4.104.320 discloses dehydrocyclization of aliphatic compounds in the presence of hydrogen using a catalyst comprising zeolite L and a group VIII metal, in which the zeolite L is of the formula:
$M9/n (AlO_2)9(SiO_2)27$

(where M is a cation of valence n) but the silica to alumina ratio may vary from 5 to 7. Zeolite L is described as occuring in the form of cylindrical crystals a few hundred Angstroms in diameter.

East German Patent 88789 discloses dehydrocyclization using a catalyst formed from a zeolite precursor with a silica to alumina ratio of up to 70. Zeolite L is mentioned as a precursor.

European Patent Application Publication 40119 discloses a dehydrocyclization process operating at low pressure (1 to 7 bars) or low $H_2$/hydrocarbon ratio using a catalyst comprising platinum on a potassium zeolite L. BE 888365 describes dehydrocyclization using a catalyst comprising platinum. rhenium (incorporated in the form of its carbonyl) and sulphur to give an atomic ratio of sulphur to platinum of 0.05 to 0.6 on a zeolitic crystalline aluminosilicate base such as zeolite L. BE792608 discloses the treatment of zeolite L for use as catalyst in isomerization by exchange with ammonium and chromium ions. EP0142351 describes a method for improving the dispersion of noble metal particles on zeolite L to provide an improved reforming catalyst. EP0145289 describes a catalyst comprising zeolite L and highly dispersed particles of a Group VIII metal. EP0142352 describes a method of regenerating a catalyst comprising zeolite L and a Group VIII metal.

GB2116450 describes a zeolite catalyst comprising a zeolite of the L family, at least one Group VIII metal and an alkaline earth metal selected selected from barium, strontium and calcium. The catalyst is used for reforming, dehydrocyclizing acyclic hydrocarbons, dehydroisomerizing alkylcyclopentanes and dealkylating toluene. Process using such catalyst are described in GB2114150 and GB2142648.

It has now been found that an improved zeolite L, has a characteristic morphology and may be valuable for use as a catalyst base in hydrocarbon conversions such as aromatization.

Thus, in one aspect this invention concerns zeolite L comprising crystallites having a hexagonal cross-section. The crystallites preferably have a mean diameter (d) of at least 0.05 $\mu$, more preferably at least 0.1 $\mu$. The crystallites are preferably elongate, and thus in the form of rods, substantially uniform along their lengths (1). The aspect ratio (the ratio of the axial length of the cylindrical surface 1 to the mean diameter d) is preferably at least 0.75 more preferably from 1 to 5.

A particularly preferred zeolite L of the invention comprises crystallites in the form of well-defined, smooth-surfaced hexagonal rods, having a mean diameter d of from 0.5 to 4$\mu$, and an aspect ratio of from 1 to 5.

The zeolite L of the invention is characterised by its crystallites having hexagonal cross-section, and the term "hexagonal" includes cross-sections in the form of a curvilinear hexagon.

The zeolite L of the invention displays an x-ray diffraction pattern typical for zeolite L, subject to the changes in position and intensity of the x-ray lines discussed in EP0096479. Occasionally, additional lines not belonging to the pattern for zeolite L appear in a pattern along with the x-ray lines characteristic of the zeolite. This is an indication that one or more additional crystalline materials are mixed with zeolite L in the sample being tested. It is a preferred feature of the invention that the amount of such additional crystalline material is minimised in the zeolite material as synthesized.

The zeolites of the invention are preferably aluminosilicates and will be described hereinafter in terms of aluminosilicates, though other elemental substitutions are possible, for example aluminium may be substituted by gallium, boron, iron and similar trivalent elements, and silicon may be substituted by elements such as germanium or phosphorus. The aluminosilicates preferably have a composition (expressed in terms of molar ratios of the constituent oxides in anhydrous form) of:

$(0.9 - 1.3)$ $M_{2/n}O:Al_2O_3:xSiO_2$

wherein M represents one or more cations of valence n, x is from 5 to 7.5, preferably from 6.5 to 7.5. The zeolitic materials of the invention have high crystallinity as shown by a well-defined x-ray diffraction pattern (without binder or other diluents present) with sharp peaks.

The exchangeable cation M in general formula I is typically potassium, but it is possible for a part of M to be replaced by other cations $M^1$ such as alkali and alkaline earth metals for example sodium, magnesium, calcium or barium. As described hereinafter, such cations may be introduced during synthesis. The ratio $M_{2/n}O:Al_2O_3$ is preferably from 0.95 to 1.15 and generally above 1.

The aluminosilicate forms of the invention may be hydrated, typically with from 0 to 9 moles of water per mole of $Al_2O_3$. When used as a catalyst base, as described hereinafter. the zeolite of the invention is preferably first calcined to remove water. In normal preparation from aqueous gels a hydrated form is first prepared and this may be dehydrated by heating.

It is a surprising feature of the invention that the zeolite L with hexagonal morphology may be prepared by ageing a synthesis gel at elevated temperatures.

In another aspect, therefore, the invention provides a process for the preparation of zeolite L crystallites having a hexagonal cross-section, in which an alkaline reaction mixture comprising water, a source of potassium, a source of silicon and a source of aluminium with a composition falling within the following molar ratios (expressed as oxides):

$M_2O/SiO_2$ 0.22- 0.36

$H_2O/M_2O$ 25 - 90

$SiO_2/Al_2O_3$ 8 - 15

(wherein M is a metal cation of valence n) is heated such that the temperature of the reaction mixture is not less than 200°C for a period long enough to form the desired hexagonal zeolite L but not so long as to produce significant amounts of non-zeolite phases.

There are four principal components to the reaction mixture or synthesis gel and thus generally:

5

aluminium
silicon
potassium
water

and the relative proportions of these components and the chosen reaction conditions are important if the desired zeolite L of the invention is to be obtained.

In addition to varying the proportions of the reactants in the reaction mixture, it is possible to vary the reaction conditions and in particular the crystallisation temperature. By using different temperatures, it may be possible to deviate further from the preferred composition defined above and yet still obtain the zeolite L. In general, within the broad reactant ratios defined for the process of the invention, a higher crystallisation temperature enables the silicon content to be lowered and/or the water content to be lowered and/or the potassium content (and thus the alkalinity) to be raised. It is a surprising aspect of the invention that the particular combination of temperature and reactant ratios specified enables a new, hexagonal form of zeolite L to be prepared.

It is preferred that the synthesis of the zeolite of the invention is conducted so that the amount of zeolite W in the product of the synthesis is minimized. Further, the synthesis of the zeolite of the invention is preferably conducted such that the product of the synthesis is substantially free of any additional crystalline non-zeolite phase such as these giving rise to a line in the x-ray pattern at d (Å) value of 6.28 ± 0.05. This may be achieved by limiting the crystallization time.

In the synthesis of all zeolitic materials of the invention, the source of silicon for the reaction mixture is generally silica, and this is usually most conveniently in the form of a colloidal suspension of silica such as Ludox HS 40 available from E.I. Dupont de Nemours and Co. Colloidal silicon sols are preferred since they result in less contaminating phases. However, other forms such as silicates may be used.

The source of aluminium may be an alumina introduced into the reaction medium as, for example, $Al_2O_3.3H_2O$, previously dissolved in alkali. However, it is also possible to introduce aluminium in the form of the metal, which is dissolved in alkali.

The aluminosilicates of the invention are preferably obtained from reaction mixtures containing potassium. This potassium is preferably introduced as potassium hydroxide.

The product of the processes described above is preferably a zeolite where M is potassium and optionally also a second metal $M^1$. By ion exchange of the product in 15 the manner conventional to zeolite chemistry, other cations can be introduced for potassium or $M^1$. It is also possible to remove any $M^1$ by ion exchange with a potassium compound such as potassium hydroxide.

Within the ranges specified hereinbefore for the composition of the reaction mixture, it is possible to choose ratios of oxides and alkalinity to given particular forms of the aluminosilicate product. The $SiO_2/Al_2O_3$ ratio in the reaction mixture may vary over a wide range but the $SiO_2/Al_2O_3$ ratio in the product preferably lies in a relatively narrow range of 5.7 to 7.4. The higher the $SiO_2/Al_2O_3$ ratio in the reaction mixture, the higher the ratio in the product. Also, decreasing alkalinity ($OH^-/SiO_2$) tends to increase the $SiO_2/Al_2O_3$ ratio in the formed product. Dilution of the reaction mixture with water and thus increasing the $H_2O/K_2O$ ratio also tends to increase the $SiO_2/Al_2O_3$ ratio in the product.

Particle size is also affected by the composition of the reaction mixture and the nature of the raw materials used. Larger particle sizes are favoured by of lower alkalinity. Higher dilution and high temperatures tend to favour formation of particles with an increased l/d ratio.

Crystallisation time is related to the crystallisation temperature. The crystallisation may be partly carried out in temperatures below 200°C, thus at temperatures of 75°C to 200°C preferably 100°C to 175°C, provided that for at least part of the time the crystallisation temperature is 200°C or greater. The period at the higher temperature will generally be from 5 hours to 72 hours, but shorter times are possible when higher temperatures are used. A time of 8 to 45 hours is typical for the period at a temperature of 200°C or greater.

The crystallisation is generally carried out in a sealed autoclave and thus at autogenous pressure. It is generally inconvenient, although possible, to employ higher pressures. Lower pressure will require longer crystallisation times.

Following the preparation as described above the zeolite L may be separated, washed and dried in the normal manner.

The products of the processes of the invention described hereinbefore are preferably substantially free from contaminant crystalline and amorphous materials. However, in employing these products in catalytic applications it may be desired to combine them with additional crystalline or amorphous materials and this invention extends to such combinations.

We have found that the zeolite L of the invention is an excellent catalyst base and may be used in a wide variety of catalytic reactions. The particular morphology of the crystals appears to result in a particular stable base for catalytically active metals with a surprising resistance to metal catalyst deactivation. In addition, the zeolite L of the invention has displayed low acidity which makes it especially suited to catalytic applications where a low acid site strength is advantageous such as aromatisation.

The catalytically-active metal(s) may be, for example, a Group VIII metal such as platinum, tin, or germanium as described in US 4,104,320, or a combination of platinum and rhenium as described in GB 2,004,764 or B E 888365. In the latter case, the catalyst may for appropriate circumstances also incorporate halogen as described in US 4,165,276, silver as described in US 4,295,959 and US 4,206,040, cadmium as described in US

0 220 881

4,295,960 and US 4,231,897 or sulphur as described in GB 1,600,927.

We have found a particularly advantageous catalyst composition to incorporate from 0.1 to 6.0 weight%, preferably from 0.1 to 1.5 weight% platinum or palladium, since this gives excellent results in aromatisation. From 0.4 to 1.2 weight% platinum is particularly preferred, especially in conjunction with the potassium form of the aluminosilicate. The invention extends to catalysts comprising the zeolitic material and a catalytically-active metal.

It may also be useful to incorporate into the catalyst of the invention one or more materials substantially inert under the conditions in which the catalyst is to be employed to act as a binder. Such binders may also act to improve the resistance of the catalyst to temperature, pressure and attrition.

The zeolite L of the invention may be used in a process for the conversion of a hydrocarbon feed in which the feed is contacted with a catalyst as described above under appropriate conditions to bring about the desired conversion. They may, for example, be useful in reactions involving aromatisation and/or dehydrocyclisation and/or isomerisation and/or dehydrogenation reaction. They are particularly useful in a process for the dehydrocyclisation and/or isomerisation of aliphatic hydrocarbons in which the hydrocarbons are contacted at a temperature of from 370 to 600°C, preferably 430 to 550°C, with a catalyst comprising zeolite L of the invention, preferably having at least 90% of the exchangeable cations M as alkali metal ions, and incorporating at least one Group VIII metal having dehydrogenating activity, so as to convert at least part of the aliphatic hydrocarbons into aromatic hydrocarbons. Preferably the invention provides a method of aromatizing a feed containing $C_6$ aliphatic hydrocarbons to form benzene.

The process is preferably otherwise carried out in the manner described in US 4,104,320, BE 888365, EP 40119, EP 0142351, EP 0145289 or EP0142352.

It has been found that use of the zeolite L of the invention as a catalyst base in this way enables hydrocarbon feeds containing $C_6$ aliphatic hydrocarbons including n-hexane to be converted to benzene with extended catalyst lifetime before deactivation as compared to conventionally prepared zeolite.

The invention will now be described in more detail, though only by way of illustration, in the following examples and evaluations.

Example 1: Preparation of Zeolite L

A synthesis gel was prepared having the following composition expressed in moles of pure oxide:
$2.62K_2O:Al_2O_3:10SiO_2:158H_2O$
This gel was prepared as follows:
The aluminium hydroxide was dissolved by boiling in an aqueous solution of potassium hydroxide pellets (86% pure KOH) to form Solution A. After dissolution any water loss was corrected. A separate solution, Solution B, was prepared by diluting colloidal silica (Ludox HS 40) with water.

Solution A and B were mixed for two minutes to form a gel, and just before the gel became fully stiff, it was transferred to a Teflon-lined autoclave, preheated to 200°C and held at that temperature for 45 hours to bring about crystallisation.

The product was a highly crystalline zeolite L (as demonstrated by X-ray diffraction) free from contamination by zeolite W and scanning electron micrographs (SEM) showed the crystallites to be rods of hexagonal cross-section having a mean diameter of about 2 microns and an aspect ratio of from 1 to 1.5.

Example 2

An identical synthesis gel to that used in Example 1 was prepared and heated in an autoclave for 20 hours at 100°C then at 225°C for 8 hours. The product was a highly crystalline zeolite L free from zeolite W. SEM showed the crystallites to be rods of hexagonal cross-section with a mean diameter of 1 micron and an aspect ratio of from 1 to 2.

Example 3

Zeolite L with hexagonal cross-section crystallites prepared according to Example 1 were washed by being formed into a 10 wt% slurry in distilled water and then stirred for 4 hours. The zeolite L was recovered and dried at 150°C overnight then impregnated with platinum by an incipient wetness technique to give 0.64 wt% pt content.

In the incipient wetness impregnation technique the dried zeolite L was added to a 1.68 wt% solution of platinum tetramine dichloride monohydrate, stirred and aged at room temperature for 30 minutes, then dried in a vacuum oven 120°C for 4 hours.

The impregnated material is pelletized and crushed to a 16-45 mesh (US sieve size) fraction. This is then calcined in air at 480°C and reduced with hydrogen at 510°C to form a catalyst ready for use.

The performance of this is evaluated in aromatisation in a high severity test designed to correlate with aromatisation performance. In this test the catalyst is placed in an isothermal microreactor at 510°C and a feed of 60% 3-methyl-pentane and 40% n-hexane is passed over the catalyst at 738 kPa, WHSV = 8.0 hr$^{-1}$ and with ratio of feed to hydrogen of 4.25. The $C_6$ conversion, benzene yield and selectivity were measured, and the results are given below for performance after 50 hours in comparison with the performance of a catalyst prepared in a similar manner but starting from cylindrical zeolite L (L = 0.5 - 0.7 micron), d = 1.0 - 1.5 micron) according to EP 0096479.

7

| | Invention | Comparison |
|---|---|---|
| Catalyst Substrate | Hexagonal Zeolite L | Cylindrical Zeolite L |
| Time on stream (hr) | 50 | 50 |
| $C_6$ conversion (%) | 53.0 | 59.4 |
| Benzene yield | 38.8 | 40.8 |
| Benzene selectivity | 73.2 | 68.6 |

A significantly higher selectivity was achieved which may be highly advantageous in some applications; from correlation established between this test and the performance test reported in EP 0096467 it is estimated that the catalyst of the invention would give a catalyst life-time of approximately 700 hr in the test of EP 0096479.

**Claims**

1 Zeolite L characterized by crystallites having a hexagonal cross-section.

2 Zeolite L as claimed in claim 1, with an aspect ratio ($_1$/d) of at least 0.75.

3 Zeolite L characterized in that the crystallites are in the form of well-defined, smooth-surfaced hexagonal rods, having mean diameter d of from 0.5 to 4 $\mu$, and an aspect ratio of from 1 to 5.

4 The use of the zeolite of any of claims 1 to 3 as a catalyst substrate in catalytic reactions.

5 A method of aromatising a $C_6$ aliphatic hydrocarbon feed to convert at least a part of feed into benzene. in which the feed is passed over a catalyst comprising zeolite L as claimed in any of claims 1 to 3 incorporating from 0.4-1.2 wt% platinum at a temperature of from 430° to 550°C.

6 A catalyst comprising zeolite L as claimed in any of claims 1 to 3 and from 0.1 to 6.0 wt% platinum or palladium.

8